(19) Europäisches Patentamt
European Patent Office
Office européen des brevets

(11) Veröffentlichungsnummer: **0 003 505**
**B1**

(12) # EUROPÄISCHE PATENTSCHRIFT

(21) Anmeldenummer: **79100105.0**

(22) Anmeldetag: **15.01.79**

(51) Int. Cl.[3]: **C 07 C 127/00,**
C 07 C 127/24

(54) **Verfahren zur Herstellung von Biuret- und/oder Harnstoffgruppen aufweisenden organischen Polyisocyanaten, sowie Dispersionen von Harnstoffgruppen aufweisenden Polyisocyanaten in Harnstoffgruppen-freiem Diisocyanat**

(30) Priorität: **25.01.78 DE 2803103**

(43) Veröffentlichungstag der Anmeldung:
**22.08.79 Patentblatt 79/17**

(45) Bekanntmachung des Hinweises auf die Patenterteilung:
**10.12.80 Patentblatt 80/25**

(84) Benannte Vertragsstaaten:
**BE DE FR GB IT NL**

(56) Entgegenhaltungen:
**DE - A - 2 261 065**
**FR - A - 2 156 375**

(73) Patentinhaber: **BAYER AG**
**Zentralbereich Patente, Marken und Lizenzen**
**D - 5090 Leverkusen 1, Bayerwerk (DE)**

(72) Erfinder: **Hennig, Hans Joachim, Dr.**
**Walter-Flex-Strasse 10**
**D - 5090 Leverkusen (DE)**
**Ziemek, Peter, Dr.**
**Berg. Landstrasse 207**
**D - 5090 Leverkusen (DE)**
**Schellmann, Erhard, Dr.**
**An der Ruten 1**
**D - 5000 Köln 80 (DE)**

**EP 0 003 505 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Verfahren zur Hestellung von Biuret- und/oder Harnstoffgruppen aufweisenden organischen Polyisocyanaten, sowie Dispersionen von Harnstoffgruppen aufweisenden Polyisocyanaten in Harnstoffgruppen-freiem Diisocyanat

Die vorliegende Erfindung betrifft ein verbessertes Verfahren zur Herstellung von Biuret- und/oder Harnstoffgruppen aufweisenden Polyisocyanaten durch Umsetzung von organischen Polyaminen.

Die Umsetzung von niedermolekularen organischen Polyisocyanaten mit niedermolekularen organischen Polyaminen sowohl zu Harnstoffen als auch zu Biureten ist bekannt. Wegen der extrem hohen Reaktionsbereitschaft von Isocyanatogruppen gegenüber primären Aminogruppen fand die Umsetzung aus den genannten niedermolekularen Ausgangsmaterialien, von einigen wenigen Ausnahmen abgesehen, keinen Eingang in die großtechnische Praxis. Dies liegt vor allem daran, daß wegen der extrem hohen Reaktionsbereitschaft der genannten Reaktivgruppen bei großtechnischen Ansätzen eine kontrollierte Umsetzung zu definierten Endprodukten kaum möglich ist.

Aus diesem Grund erfolgt bislang auch die Herstellung von Biuretpolyisocyanaten in der Großtechnik vorzugsweise durch Umsetzung von organischen Diisocyanaten mit "Biuretisierungsmitteln", d.h. Verbindungen wie beispielsweise Wasser, welche mit Isocyanatgruppen in einem ersten Reaktionsschritt zunächst zu Aminogruppen abreagieren, worauf sich die Biuretisierungsreaktion zwischen dem in situ gebildeten Amin und überschüssigem Isocyanat anschließt (DT—PS 1 101 394). Da bei diesem Verfahren die Aminogruppen niemals in nennenswerter Konzentration vorliegen, treten die auf die hohe Reaktivität zurückzuführenden negativen Begleiterscheinungen nicht auf.

Das Verfahren der GB—PS 1 263 609 beschreibt eine weitere Methode, die auf die hohe Reaktivität zurückzuführenden Schwierigkeiten zu überwinden, welche darin besteht, daß man die Diisocyanate nicht mit den freien Diaminen zur Reaktion bringt, sondern vielmehr durch Zusatz von Carbonylverbindungen zu den Aminen die Konzentration der hochreaktiven Aminogruppen vermindert.

Die direkte Reaktion zwischen niedermolekularen diprimären Diaminen und niedermolekularen Diisocyanaten zu den entsprechenden Biuretpolyisocyanaten wird in der DT—OS 2 261 065 bzw. der US—PS 3 903 126 beschrieben. Jedoch zeigt ein Nacharbeiten der Ausführungsbeispiele dieser Veröffentlichung, daß nur die speziellen, als bevorzugt bezeichneten Diamine zur Herstellung von technisch brauchbaren Biuretpolyisocyanaten geeignet sind, während das wichtigste aliphatische Diamin, Hexamethylendiamin, insbesondere in Kombination mit Hexamethylendiisocyanat nach dem Verfahren der DT—OS 2 261 065, wie aufgrund der obigen Ausführungen zu erwarten, nicht zu einem sedimentfreien und hellfarbigen Biuretpolyisocyanat umgesetzt werden kann.

Das Verfahren der DT—OS 2 609 995 gestattet zwar die Herstellung von derartigen hellfarbigen sedimentfreien Biuretpolyisocyanaten durch direkte Umsetzung von Hexamethylendiamin mit Hexamethylendiisocyanat, jedoch ist das Verfahren dieser Veröffentlichung mit dem Nachteil verbunden, daß das Diamin gasfömig in das vorgelegte Diisocyanat eingeleitet werden muß, was einen zusätzlichen Aufwand darstellt.

Die Umsetzung zwischen organischen Polyisocyanaten insbesondere Diisocyanaten mit diprimären organischen Diaminen zu den entsprechenden Harnstoffisocyanaten fand schließlich überhaupt noch keinen Eingang in die großtechnische Praxis. Die Bildung derartiger Harnstoffisocyanate wird lediglich in den genannten Veröffentlichungen als unerwünschte Begleiterscheinung bei der Herstellung von Biuretpolyisocyanaten erwähnt, ohne das es bislang gelungen wäre, die direkte Reaktion zwischen diprimären organischen Diaminen und überschüssigen Mengen an niedermolekularen organischen Diisocyanaten zu den entsprechenden Harnstoffgruppen aufweisenden Polyisocyanaten einer technisch sinnvollen Nutzung zuzuführen.

Durch das nachstehend beschriebene erfindungsgemäße Verfahren wird nun erstmals ein Weg gewiesen, beliebige, mindestens zwei primäre Aminogruppen aufweisende organische Verbindungen mit beliebigen organischen Polyisocyanaten in direkter Reaktion zu dem entsprechenden Harnstoffgruppen bzw. Biuretgruppen aufweisenden Polyisocyanaten umzusetzen, ohne auf die Verwendung spezieller Diamine, die Verwendung spezieller Hilfsmittel (beispielsweise Ketone) bzw. ein gasförmiges Einleiten des Diamins angeweisen zu sein. Beim nachstehend beschriebenen erfindungsgemäßen Verfahren kann die Umsetzung durch einfache Wahl der Reaktionstemperatur so gesteuert werden, daß wahlweise Lösungen der entsprechenden Biuretpolyisocyanate in überschüssigem Polyisocyanat oder sedimentstabile Dispersionen der entsprechenden Harnstoffpolyisocyanate in überschüssigem Polyisocyanat erhalten werden. Das erfindungsgemäße Verfahren gestattet daher nicht nur auf besonders einfache Weise die Herstellung von an sich bekannten Biuretpolyisocyanaten, wie beispielsweise solchen auf Basis von Hexamethylendiamin und Hexamethylendiisocyanat, sondern vielmehr auch erstmals die Herstellung von technisch äußerst interessanten Dispersionen von Harnstoffdiisocyanaten in überschüssigem Diisocyanat. Derartige sedimentstabile Dispersionen sind bislang nicht bekannt

geworden. Sie stellen für den Polyurethanchemiker besonders interessante Ausgangsmaterialien dar.

Gegenstand der vorliegenden Erfindung ist ein Verfahren zur Herstellung von Harnstoffgruppen und/oder Biuretgruppen aufweisenden Polyisocyanaten in in Harnstoff- und Biuretgruppen-freien Polyisocyanaten dispergierter bzw. gelöster Form durch Umsetzung von organischen Polyisocyanaten mit mindestens 2 primäre Aminogruppen aufweisenden organischen Polyaminen in einem $NCO/NH_2$-Äquivalentverhältnis von mindestens 4:1, wobei das sowohl als Reaktionspartner als auch als Lösungs- bzw. als Dispergiermittel dienende, im Überschuß zur Anwendung gelangende Polyisocyanat vorgelegt und das im Unterschuß zum Einsatzgelangende Polyamin in das vorgelegte Polyisocyanat bei einer Temperatur im Temeperaturbereich von —20 bis 250°C eingebracht wird, wobei die Herstellung der Biuretgruppen aufweisenden Polyisocyanate gegebenenfalls zweistufig dergestalt erfolgt, daß man eine zunächst bei relativ tiefer Temperatur gebildete Dispersion von Harnstoffgruppen aufweisenden Polyisocyanaten durch Nacherhitzen in die Lösung von Biuretgruppen aufweisenden Polyisocyanaten überführt, dadurch gekennzeichnet, daß man

a) die im Überschuß zum Einsatz gelangende Polyisocyanatkomponente in einem Mischraum vorlegt, dessen Abmessungen dergestalt sind, daß die Entfernung von der nachstehend unter b) genannten Düse zur Innenwand des Mischraums in Richtung des eingedüsten Strahls mindestens das 100-fache des Düsendurchmessers und die kürzeste Entfernung des Düsenstrahls zur seitlichen Begrenzungsfläche des Mischraums mindestens das 25-fache des Düsendurchmessers beträgt, und

b) die im Unterschuß zum Einsatz gelangende Polyaminkomponente mittels einer Glattstrahldüse mit einem lichten Durchmesser von 0,01 bis 5 mm unter einem Druck von 2 bis 1000 bar und mit einer Relativgeschwindigkeit von mindestens 5 m/s in die vorgelegte Polyisocyanatkomponente eindüst.

Gegenstand der vorliegenden erfindung ist auch ein Verfahren zur Herstellung von Monomeren-freien Polyisocyanaten, welches dadurch gekennzeichnet ist, daß man aus den gemäß obigem Verfahren erhältlichen Lösungen von Biuretgruppen aufweisenden Polyisocyanaten in Harnstoff- und Biuretgruppen-freien Polyisocyanaten diese durch Abdestillieren oder Extrahieren entfernt.

Gegenstand der vorliegenden Erfindung sind schließlich auch stabile Dispersionen von Harnstoffgruppen aufweisenden Polyisocyanaten der Formel

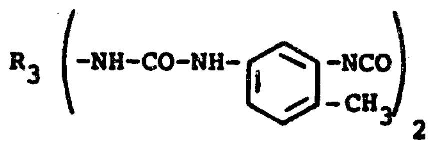

in 2,4- und/oder 2,6-Diisocyanatotoluol, wobei $R_3$ für den 2-wertigen Rest steht, wie er durch Entfernung der Aminogruppen aus einem gegebenenfalls Methyl-substituierten und/oder Methylenbrücken aufweisenden aromatischen diprimären Diamin mit insgesamt 6 bis 15 Kohlenstoffatomen erhalten wird.

Ausgangsmaterialien beim erfindungsgemäßen Verfahren sind beliebige organische Polyisocyanate. Vorzugsweise werden beim erfindungsgemäßen Verfahren organische Diisocyanate eingesetzt. Geeignete Diisocyanate sind insbesondere solche der Formel

$$R_1(NCO)_2$$

in welcher

$R_1$ für einen aliphatischen Kohlenwasserstoffrest mit 2 bis 18 Kohlenstoffatomen, einen aromatischen Kohlenwasserstoffrest mit 6 bis 15 Kohlenstoffatomen, einen araliphatischen Kohlenwasserstoffrest 8 bis 15 Kohlenstoffatomen oder einen cycloaliphatischen Kohlenwasserstoffrest mit 4 bis 15 Kohlenstoffatomen steht, wobei jeweils zwischen den beiden Isocyanatgruppen mindestens 2 Kohlenstoffatome angeordnet sind.

Beispiele derartiger Diisocyanate sind Äthylendiisocyanat, Hexamethylendiisocyanat, Decamethylendiisocyanat, Undecamethylendiisocyanat, Octadecamethylendiisocyanat, 3,3,5-Trimethyl-1,6-diisocyanatohexan, p-Phenylendiisocyanat, 2,4-Diisocyanatotoluol, 2,6-Diisocyanatotoluol, 4,4'-Diisocyanatodiphenylmethan, 2,4'-Diisocyanatodiphenylmethan, Gemische der beiden letztgenannten Isomeren mit ihren höherkernigen Homologen, wie sie bei der an sich bekannten Phosgenierung von Anilin/Formaldehyd-Kondensaten anfallen, p-Xylylendiisocyanat, Cyclobutan-diisocyanat-1,3, 1,4-Diisocyanato-cyclohexan, 1-Methyl-2,4-diisocyanato-cyclohexan, 4,4"-Diisocyanato-dicyclohexylmethan, 3,3,5-Trimethyl-5-isocyanatomethyl-cyclohexylisocyanat (IPDI), sowie Gemische dieser Diisocyanate.

Bevorzugte Diisocyanate für das erfindungsgemäße Verfahren sind 2,4-Diisocyanatotoluol, dessen technische Gemische mit 2,6-Diisocyanatotoluol, 4,4'-Diisocyanatodiphenylmethan, dessen technischen Gemische mit 2,4'-Diisocyanatodiphenylmethan, Hexamethylendiisocyanat und IPDI. Besonders bevorzugte Diisocyanate sind die genannten Diisocyanatotoluole bzw. Hexamethylendiisocyanate.

Reaktionspartner für die beispielhaft genannten Polyisocyanate sind beliebige mindestens 2 primäre Aminogruppen aufweisende organische Verbindungen, die

vorzugsweise von den Aminogruppen abgesehen gegenüber Isocyanatgruppen inert sind. Besonders bevorzugt werden beim erfindunggemäßen Verfahren diprimäre Diamine der Formel

$$R_2(NH)_2)_2$$

eingesetzt, wobei

$R_2$ gleich oder verschieden von $R_1$ ist und im übrigen der Definition von $R_1$ entspricht.

Für das erfindungsgemäße Verfahren sind dementsprechend insbesondere die den beispielhaft genannten Diisocyanaten entsprechenden Diamine geeignet.

Bevorzugte beim erfindungsgemäßen Verfahren einzusetzende Diamine sind 2,4-Diaminotoluol, dessen technischen Gemische mit 2,6-Diaminotoluol, 4,4'-Diaminodiphenylmethan, dessen technische Gemische mit 2,4'-Diaminodiphenylmethan, Hexamethylendiamin, 4,4' - Diaminodicyclohexylmethan und seine Homologen, sowie 3,3,5-Trimethyl-5-aminomethylcyclohexylamin (IPDA). Besonders bevorzugte Diamine sind die genannten Diaminotoluole, Diaminodiphenylmethane und Hexamethylendiamin.

Erfindungswesentlicher Punkt ist die Verwendung einer ganz bestimmten Düse in Kombination mit einem ganz bestimmten Mischraum. Das erfindungsgemäße Verfahren wird unter Verwendung von Glattstrahldüsen des Durchmessers 0,01 bis 5, vorzugsweise 0,1 bis 1 mm durchgeführt, wobei die im Überschuß sum Einsatz gelangende Isocyanat-Komponente vorgelegt und die im Unterschuß zum Einsatz gelangende Aminkomponente in die vorgelegte Isocyanatkomponente eingedüst wird. Hierbei ist darauf zu achten, daß die eingedüste Komponente mindestens mit einer Differenzgeschwindigkeit von 5 m/sec in die vorgelegte Komponente eintritt und daß der Mischraum so dimensioniert ist, daß die Entfernung von der Düse in Richtung des eingedüsten Strahls bis zur, der Düse gegenüberliegenden Mischraum-Wandung mindestens das 100-fache und die kleinste seitliche Entfernung vom Strahl zur Innenwand des Mischraums mindestens das 25-fache des Düsendurchmessers beträgt. Bei der Durchführung des erfindungsgemäßen Verfahrens ist ferner darauf zu achten, daß der Druck in der Mischkammer stets größer ist als der unter den jeweiligen Temperaturbedingungen vorherrschende Dampfdruck der am leichtesten flüchtigen, im Mischraum befindlichen Komponente, so daß Dampf- oder Gasblasen im Mischraum nie auftreten. Die einzudüsende Komponente wird unter einem Druck von 2 bis 1000 bar, vorzugsweise 10 bis 200 bar, eingedüst. Die Ausbildung eines unerwünschten Gasraums am Düsenaustritt und in der Mischzone kann auch durch ein Eindüsen von unten nach oben verhindert werden, da so eventuell trotz der gewählten Druckbedingungen entstehende Gasblasen nach oben aus der Mischzone entweichen würden.

Figur 1 zeigt eine für das erfindungsgemäße Verfahren geeignete Glattstrahldüse. Hierbei bedeuten

(1) den Eingang zur Düse und

(2) die eigentliche Glattstrahldüse.

Figur 2 zeigt eine denkbare Vorrichtung zur kontinuierlichen Durchführung des erfindungsgemäßen Verfahrens. Hierbei bedeuten

(1) ein Vorratsbehälter für die Isocyanatkomponente,

(2) eine Pumpe für das Isocyanat,

(3) die Düse,

(4) den Mischraum,

(5) den Vorratsbehälter für Verfahrensprodukt,

(6) den Vorratsbehälter für eine Spülflüssigkeit,

(7) eine Hochdruckpumpe und

(8) ein Vorratsgefäß für die Aminkomponente.

Bei der kontnuierlichen Durchführung des erfindungsgemäßen Verfahrens in einer der Figur 2 entsprechenden Vorrichtung empfiehlt sich, bei der Inbetriebnahme vor Zusammentreffen der Reaktionspartner den vollen Betriebsdruck aufzubauen, so daß sofort zu Beginn der erfindungsgemäßen Reaktion die erforderliche Ausströmgeschwindigkeit gewährleistet ist. Es hat sich hierfür als voreteilhaft erwiesen, vor Beginn der Hochdruckverdüsung die Zufuhrleitung zur Glattstrahldüse mit einer inerten Flüssigkeit zu füllen, damit während der Zeitspanne, in der ein Druckaufbau bis auf Betriebsdruck erfolgt, an der Düse lediglich inerte Flüssigkeit austritt.

Bei der Durchführung des erfindungsgemäßen Verfahrens werden die Reaktionspartner in einem $NCO/NH_2$-Äquivalentverhältnis von 4:1 bis 1000:1, vorzugsweise 5 zu 1 bis 25 zu 1 zur Umsetzung gebracht. Bei der kontiunierlichen Durchführung des erfindungsgemäßen Verfahrens ensspricht die Menge der kontinuierlich in die Mischkammer eingebrachten Reaktionspartner dem vorgewählten Äquivalentverhältnis innerhalb der genannten Bereiche. Bei der ebenfalls möglichen diskontiunierlichen Durchführung des erfindungsgemäßen Verfahrens wird die Gesamtmenge der Isocyanatkomponente in einem geeigneten Reaktionsgefäß, beispielsweise einem Reaktionskessel, vorgelegt, während die Düse in die im Reaktionsgefäß befindliche Flüssigkeit eintaucht.

Durch einfache Wahl der Ausgangskomponenten, sowie der Reaktionstemperatur kann das erfindungsgemäße Verfahren so gesteuert werden, daß als Verfahrensprodukte sedimentstabile Dispersionen von Harnstoffgruppen aufweisenden Diisocyanaten in überschüssigem Ausgangsisocyanat oder Lösungen von Biuretgruppen aufweisenden Polyisocyanaten in überschüssigen Ausgangsisocyanat erhalten werden. So entstehen beim erfindungsgemäßen Verfahren feindisperse Harnstoffdispersionen mit einer mittleren Teilchengröße von ca. 0,5 bis 250 $\mu$m, wenn die Temperatur im Reaktionsraum unterhalb der Schmelztemperatur des sich stets primär aus Isocyanat und Amin bildenden Harnstoffs gehalten wird. Die Herstellung von Lösungen von Biuretgruppen aufweisenden Polyisocyanaten un überschüssigem Ausgangsisocyanat kann entweder 2-stufig durch Nacherhitzen der zunächst bein relativ tieferer Temperatur gebildeten Harnstoffdispersionen oder aber auch dadurch erfolgen, daß man bereits während der Durchführung des erfindungsgemäßen Verfahrens die Temperatur im Reaktionsgefäß so einstellt, daß der sich zunächst bildende Harnstoff sofort als im Ausgangsisocyanat lösliche und mit dem überschüssigen Isocyanat zu Biuretgruppen aufweisendem Polyisocyanat weiterreagierende Flüssigkeit anfällt, ohne daß makroskopisch die intermediäre Bildung des Harnstoffs überhaupt erkennbar würde. Die Temperatur, die während der Durchführung des erfindungsgemäßen Verfahrens im Reaktionsgefäß aufrechterhalten werden muß, um zu Dispersionen von Harnstoffgruppen aufweisenden Polyisocyanaten bzw. zu Lösungen von Biuretgruppen aufweisenden Polyisocyanaten in überschüssigem Ausgangsisocyanat zu gelangen kann sich in Abhängigkeit von der Natur der gewählten Ausgangsmaterialien innerhalb weiter Grenzen im Temperaturbereich von −20 bis +250°C bewegen und ist auf einfache Weise durch einen orientierenden Vorversuch zuverlässig zu ermitteln.

Das erfindungsgemäße Verfahren ist insbesondere zur Herstellung von Dispersionen von Harnstoffpolyisocyanaten auf Basis aromatishcher Diisocyanate und aromatischer Diamine in überschüssigem aromatischem Diisocyanat, sowie zur Herstellung von Biuretpolyisocyanaten mit aliphatisch gebundenen Isocyanatgruppen auf Basis von aliphatischen Diisocyanaten und aliphatischen Diaminen von Interesse.

Das erfindungsgemaße Verfahren eignet sich insbesondere zur Herstellung von Dispersionen von Harnstoffgruppen aufweisenden Diisocyanaten in 2,4-Diisocyanatotoluol bzw. dessen technischen Gemische mit 2,6-Diisocyanatotuluol. Zur Herstellung dieser Dispersion werden die zuletztgenennten Diisocyanato-toluole als Ausgangsisocyanat vorgelegt und vorzugsweise 2,4-Diaminotoluol bzw. dessen technischen Gemische mit 2,6-Diaminotoluol oder auch ein anderes aromatisches Diamin wie z.B. 4,4′-Diaminodiphenylmethan bzw. dessen technischen Gemische mit 2,4′-Diaminophenylmethan und/oder seinen höheren Homologen als Aminkomponente verwendet. Die erfindungsgemäße Umsetzung erfolgt dabei vorzugsweise im Bereich von 20 bis 120°C. Hierbei wird diese Temperatur durch geegnete Wahl der Diisocyanatkomponente und deren Temperatur unter Berücksichtigung der Wärmetönung und der Temperatur der eingedüsten Diamin-Komponente eingestellt.

Da bei der erfindungsgemäßen Umsetzung die Diisocyanat-Komponente stets im Überschuß vorleigt und da auch bei Verwendung von Gemischen von 2,4- und 2,6-Diisocyanatotoluol davon ausgegangen werden kann, daß die zur Methylgruppe p-ständige Isocyanatgruppe des 2,4-Diisocyanatotoluols bevorzugt abreagiert, entstehen bei der erfindungsgemäßen Umsetzung vorzugsweise nicht kettenverlängerte Harnstoffdiisocyanate der Formel

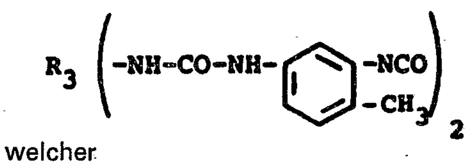

in welcher

$R_3$ für einen zweiwertigen aromatischen Kohlenwasserstoffrest steht, der gegebenenfalls Methylsubstituenten bzw. Methylenbrücken tragen kann und insgesamt 6 bis 15 Kohlenstoffatome aufweist.

Entsprechend den oben gemachten Ausführungen steht $R_3$ vorzugsweise für einen Rest, der durch Entfernung der Aminogruppen aus 2,4-Diaminotoluol, dessen technischen Gemische mit 2,6-Diaminotoluol bzw. aus 4,4′-Diaminodiphenylmethan bzw. dessen technischen Gemische mit 2,4′-Diaminodiphenylmethan erhalten wird. Bei Verwendung von Gemischen von 4,4′-Diaminodiphenylmethan (und gegebenenfalls 2,4′-Diaminodiphenylmethan) mit höhren Polyaminen der Diphenylmethanreihe, wie sie bei der an sich bekannten Anilin/Formaldehyd-Kondensation als großtechnische Produkte anfallen, entstehen die Harnstoffdiisocyanate der obengenannten Formel gleichzeitig neben der Funktionalität der höheren Homologen entsprechenden, höherfunktionellen Harnstoffpolyisocyanaten.

Die beim erfindungsgemäßen Verfahren entstehenden Dispersionen der Harnstoffdiisocyanate der zuletzt genannten Formel können als modifiziertes Toluylendiisocyanat angesehen werden, für welche sich eine Reihe äußerst interessanter Einsatzgebeite eröffnen. Im allgemeinen werden die Mengenverhältnisse bei der Herstellung dieser Dispersionen so gewältnisse bein der Herstellung dieser Dispersionen so gewähalt, daß 5 bis 40 gew.-%ige Dispersionen der Harnstoffdiisocyanate in überschüssigem Diisocyanatotoluol entstehen, d.h. man arbeitet vorzugsweise in einem $NCO/NH_2$-Äquivalentverhältnis von ca. 50:1 bis 5:1.

Das erfindungsgemäße Verfahren eignet sich auch besonders gut zur Herstellung von Biuretgruppen aufweisenden Polyisocyanaten bzw. deren Lösungen in überschüssigem aliphatischem Diisocyanat. Hierzu dienen beim erfindungsgemäßen Verfahren als Ausgangsmaterialien vorzugsweise solche Diisocyanate der Formel

$$R_1(NCO)_2$$

$R_1$ für einen Polymethylenrest mit 4 bis 11 Kohlenstoffatomen, vorzugsweise für einen Hexamethylenrest steht.

Als Diaminkomponente kommen beim erfindungsgemäßen Verfahren zur Herstellung der Biuretgruppen aufweisenden Polyisocyanate vorzugsweise solche Diamine der Formel

$$R_2(NH_2)_2$$

zum Einsatz, für welche $R_2$ gleich oder verschieden von $R_1$ ist und ebenfalls einen Polymethylenrest mit 4 bis 11 Kohlenstoffatomen, vorzugsweise einen Hexamethylenrest, steht.

Wie bereits oben ausgeführt gelingt die Herstellung von Biuretgruppen aufweisenden Polyisocyanaten sowohl nach dem Zweistufenprinzip, d.h. intermediäre Darstellung der entsprechenden Harnstoffdispersion und deren thermische Nachbehanlung, als auch nach dem Einstufenprinzip durch Arbeiten bei entsprechend höheren Temperaturen. Bei der Herstellung der Biuretgruppen aufweisenden Polyisocyanate nach dem Zweistufenprinzip werden die beispielhaft genannten Ausgangsmaterialien vorzugsweise in einem $NCO/NH_2$-Äquivalentverhältnis von 25:1 bis 8:1 unter Aufrechterhaltung einer Reaktionstemperatur von vorzugsweise ca. 50 bis 180°C zur Umsetzung gebracht. Die dabei entstehende Harnstoffdispersion, die im übrigen auch für andere Zwecke, beispielsweise als modifiziertes aliphatisches Diisocyanat, zu verwenden ist, kann durch Erhitzen auf ca. 150 bis 250°C dann in eine hellfarbige sedimentfreie Lösung des entsprechenden Biuertgruppen aufweisenden Polyisocyanats in überschüssigem Diisocyanat überfuhrt werden. Eine derartige Lösung entsteht beim erfindungsgemäßen Verfarhen direkt ohne makroskopisch erkennbare intermediäre Bildung der genannten Harnstoffdispersion, wenn eine Reaktionstemperatur von ca. 180 bis 250°C aufrechterhalten wird. Durch Abdestillieren bzw. Extrahieren des überschüssigen Diisocyanats entstehen dann aus den Lösungen die entsprechenden Monomeren-freien Biuretpolyisocyanate, die bekanntlich äußerst wertvolle Ausgangsmaterialien zur Herstellung von Polyurethanlacken darstellen.

Das erfindungsgemäße Verfahren ist selbstverständlich nicht auf die Herstellung der als besonders bevorzugt bezeichneten Harnstoffdispersionen bzw. Biuretlösungen beschränkt, sondern eignet sich auch beispielsweise zur Herstellung von Lösungen von Biuretgruppen aufweisenden Polyisocyanaten mit aromatisch gebundenen Isocyanatgruppen nach den dargelegten Prizipien, wobei lediglich die geeignete Temperatur, je nach dem ob nach dem Einstufen- oder Zweistufenverfahren gearbeitet werden soll, durch einen kurzen Vorversuch ermittelt werden muß.

Wie bereits kurz dargelegt, ist diese Reaktionstemperatur eine Funktion sowohl der Temperatur der Isocyanatkomponente als auch der Temperatur der oft vor der Durchführung des erfindungsgemäßen Verfahrens aufzuschmelzenden Aminkomponente als auch eine Funktion der Wärmetönung der exothermen erfindungsgemäßen Umsetzung.

Die nachstehenden Beispiele dienen zur näheren Erläuterung des erfindungsgemäßen Verfahrens.

Beispiel 1

2000 l/h = 1948 kg/l auf 100°C vorerhitztes Hexamethylendiisocyanat werden in einer Vorrichtung gemäß Figur 2 kontinuierlich aus einem Vorratsbehälter (1) über eine Pumpe (2) unter einem Druck von 1,5 bar in eine zylindrische Mischkammer (4) mit einem Innendurchmesser von 10 cm und einer Länge von 30 cm gefördert. Gleichzeitig wird in die Mischkammer mittels einer Glattstrahldüse (3) mit einem Durchmesser von 0,5 mm zentral 80 kg/h auf 60°C erwärmtes Hexamethylendiamin entsprechend einem $NCO/NH_2$-Äquivalentverhältnis von 17:1 unter einem Druck (vor der Düse) von 80 bar eingedüst. Unter Erwärmen auf 140°C entsteht monentan und spontan eine feinteilige Dispersion des den Ausgangsmaterialien entsprechenden Harnstoffdiisocyanats in überschüssigem Hexamethylendiisocyanat. Die Dispersion wird kontinuierlich in das Vorratsgefäß (5) abgeführt. Die mittlere Teilchengröße des dispergierten Harnstoffdiisocyanats beträgt 15 $\mu$m, die Dispersion zeigt auch bei 10-tägigem Stehen bei Raumtemperatur keinerlei Tendenz zur Sedimentbildung. Beim Erhitzen der Dispersion auf 180°C während einer Stunds entsteht eine klare Lösung des entsprechenden Biuretgruppen aufweisenden Polyisocyanats in überschüssigem Hexamethylendiisocyanat.

Beispiel 2

Unter Verwendung der gleichen Vorrichtung wie die in Beispiel 1 wird ein Isomerengemisch aus 70 Gew.-Teilen 4,4'-und 30 Gew.-Teilen 2,4'-Diaminodiphenylmethan, das zuvor auf 100°C erwärmt wurde, unter einem Druck von 80 bar mit einem 35°C warmen Isomerengemisch aus 65 Gew.-Teilen 2,4-und 35 Gew.-Teilen 2,6-Diisocyanatotoluol im Verhältnis von 3,3 zu 96,7 Gew.-%, was einem Molverhältnis von 1:33,7 entspricht, zur Reaktion gebracht. Der Gesamtmengendurchsatz beträgt 2466 kg pro Stunde. Es entsteht eine feinteilige Polyharnstoff-Dispersion in überschüssigem Diisocyanatotoluol mit einem Feststoffgehalt von 9%, die sich durch thixotrope Eigenschaften auszeichnet. Der berechnete Isocyanat-Gehalt beträgt 45,3%; gefunden wurden 45,2%.

Erwärmt man diese Dispersion eine halbe Stunde lang auf 140°C, so entsteht eine klare Biuret-haltige Lösung mit einem NCO-Gehalt von 44,2% (berechnet 43,9%).

Beispiel 3

Mit der in Beispiel 1 genannten Verdüsungseinrichtung wurden in kontinuierlicher Verfahrensweise pro Stunde 75 kg Isophorondiamin unter einem Druck von 75 bar mit 400 kg Diisocyanatotoluol (Isomerengemisch aus 65% 2,4- und 35% 2,6-Isomeren) entsprechend einem $NCO/NH_2$-Äquivalentverhältnis von 8,46:1 bei einer Temperatur von 26° umgesetzt. Es entsteht eine Dispersion des Diisocyanato-bis-harnstoffs aus 2 Mol Diisocyanatotoluol und 1 Mol Isophorodiamin in überschüssigem Diisocyanato-toluol. Der Isocyanat-Gehalt der Dispersion beträgt 32,1%.

Bei kurzzeitigem Erhitzen der Dispersion auf 170° findet unter Minderung des Isocyanat-Gehaltes auf 25,2% Umwandlung in eine homogene klare Lösung von Biuret-polyisocyant in Diisocyanatotoluol statt. Das bei Raumtemperatur harzige Material löst sich spielend in Chlorbenzol.

Beispiel 4

Im Verlauf von 11 Minuten wurden mit der in Beispiel 1 genannten Vorrichtung 13 kg Diamino-toluol (65% 2,4-und 35% 2,6-Isomeres) unter einen Druck von 78 bar mit 648 kg Diisocyanato-toluol (65:35-Isomerengemisch) entsprechend einem $NCO/NH_2$-Äquivalentverhältnis von 35,1:1 zur Reaktion gebracht, wobei die Temperatur im Bereich der Reaktionszone von 28 auf 40°C anstieg. Erhalten wurde eine bräunliche thixotrope Dispersion mit einem Isocyanat-Gehalt von 45,2% (ber. 46,0%).

**Patentansprüche**

1. Verfahren zur Herstellung von Harnstoffgruppen und/oder Biuretgruppen aufweisenden Polyisocyanaten in in Harnstoffund Biuretgruppen-freien Polyisocyanaten dispergierter bzw. gelöster Form durch Umsetzung von organischen Polyisocyanaten mit mindestens 2 primäre Aminogruppen aufweisenden organischen Polyaminen in einem $NCO/NH_2$-Äquivalentverhältnis von mindestens 4:1, wobei das sowohl als Reaktionspartner als auch als Lösungs-bzw. Dispergiermittel dienende, im Überschuß zur Anwendung gelangende Polyisocyanat vorgelegt und das im Unterschuß zum Einsatz gelangende Polyamin in das vorgelegte Polyisocyanat bei einer Temperatur im Temperaturbereich von –20 bis 250°C eingebracht wird, wobei die Herstellung der Biuretgruppen aufweisenden Polyisocyanate gegebenenfalls zweistufig dergestalt erfolgt, daß man eine zunächst bei relativ tiefer Temperatur gebildete Dispersion von Harnstoffgruppen aufweisenden Polyisocyanaten durch Nacherhitzen in die Lösung von Biuretgruppen aufweisenden Polyisocyanaten überführt, dadurch gekennzeichnet, daß man

a) die im Überschuß zum Einsatz gelangende Polyisocyanatkomponente in einem Mischraum vorlegt, dessen Abmessungen dergestalt sind, daß die Entfernung von der nachstehend unter b) genannten Düse zur Innenwand des Mischraums in Richtung des eingedüsten Strahls mindestens das 100-fache des Düsendurchmessers und die kürzeste Entfernung des Düsenstrahls zur seitlichen Begrenzungsfläche des Mischraums mindestens das 25-fache des Düsendurchmessers beträgt, und

b) die im Unterschuß zum Einsatz gelangende Polyaminkomponente mittels einer Glattstrahldüse mit einem lichten Durchmesser von 0,01 bis 5 mm unter einem Druck von 2 bis 1000 bar und mit einer Relativgeschwindigkeit von mindestens 5 m/s in die vorgelegte Polyisocyanatkomponente eindüst.

2. Verfahren gemäß Anspruch 1, dadurch gekennzeichnet, daß man als organisches Polyisocyanat ein Diisocyanat der Formel

$$R_1(NCO)_2$$

und als Polyamin ein Diamin der Formel

$$R_2(NH_2)_2$$

verwendet, wobei

$R_1$ und $R_2$ für gleiche oder verschiedene Polymethylenreste mit jeweils 4 bis 11 Kohlenstoffatomen stehen.

3. Verfahren zur Herstellung von Monomeren-freien Biuretpolyisocyanaten gemäß Anspruch 1, dadurch gekennzeichnet, daß man aus den Losungen der Biuretgruppen aufweisenden Polyisocyanaten in Harnstoff- und Biuretgruppen-freien Polyisocyanaten die Harnstoff- und Biuretgruppen-freien Polyisocyanate durch Abdestillieren oder Extrahieren entfernt.

4. Verfahren zur Herstellung von Harnstoffgruppen aufweisenden Polyisocyanaten in in Harnstoff- und Biuretgruppen-freien Polyisocyanaten dispergierter Form gemäß Anspruch 1, dadurch gekennzeichnet, daß man als organisches Polyisocyanat 2,4-Diisocyanatotoluol und/oder 2,6-Diisocyanatotoluol und als organisches Polyamin ein aromatisches, gegebenenfalls Methyl-Substituenten oder Methylenbrücken aufweisendes diprimäres Diamin mit ingesamt 6 bis 15 Kohlenstoffatomen verwendet und man die Umsetzung im Temperaturbereich von 20 bis 120°C durchführt.

5. Stabile Dispersionen von Harnstoffgruppen aufweisenden Polyisocyanaten der Formel

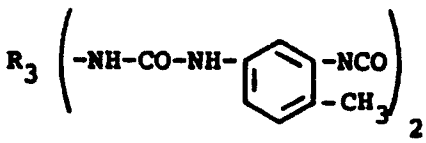

in 2,4- und/oder 2,6-diisocyanatotoluol, wobei $R_3$ für den 2-wertigen Rest steht, wie er durch Entfernung der Aminogruppen aus einem gegebenenfalls Methyl-substituierten und/oder Methylenbrücken aufweisenden aromatischen diprimären Diamin mit insgesamt 6 bis 15 Kohlenstoffatomen erhalten wird.

## Revendications

1. Procédé de production de polyisocyanates porteurs de groupes urée et/ou de groupes biuret sous une forme dispersée ou dissoute dans des polyisocyanates dépourvus de groupes urée et biuret, par réaction de polyisocyanates organiques avec des polyamines organiques portant au moins deux groupes amino primaires dans un rapport d'équivalents $NCO/NH_2$ d'au moins 4:1, dans lequel le polyisocyanate servant à la fois de partenaire réactionnel et de solvant ou de dispersant, utilisé en excès, est introduit au début de l'operation et la polyamine utilisée en quantité déficitaire est introduite dans le polyisocyanate disposé préalablement, à une température comprise dans la plage de −20 à 250°C, la production des polyisocyanates porteurs de groupes biuret s'effectuant éventuellement en deux étapes de telle manière qu'on transforme une dispersion, formée tout d'abord à une température relativement basse, de polyisocyanates porteurs de groupes urée, par chauffage subséquent, en la solution de polyisocyanates porteurs de groupes biuret, caractérisé en ce que:

a) on introduit tout d'abord le composant polyisocyanate utilisé en excès dans une chambre de mélange dont les dimensions sont choisies de manière que la distance de la buse mentionnée en b) ci-après à la paroi intérieure de la chambre de málange dans la direction du jet projeté s'élève au moins á 100 fois le diamètre de la buse et que la distance la plus courte du jet de la buse à surface latérale de délimitation de la chambre de mélange s'élève au moins à 25 fois le diamètre de la buse, et

b) le composant polyaminé utilisé en quantité déficitaire est injecté au moyen d'une buse à jet en nappe de diamètre intérieur compris entre 0,01 et 5 mm sous une pression de 2 à 1000 bars (0,2 à 100 MPa) et à une vitesse relative d'au moins 5 m/s dans le composant polyisocyanate introduit au préalable.

2. Procédé suivant la revendication 1, caractérisé en ce qu'on utilise comme polyisocyanate organique un diisocyanate de formule:

$$R_1(NCO)_2$$

et comme polyamine, une diamine de formule:

$$R_2(NH_2)_2$$

$R_1$ et $R_2$ représentant des restes polyméthyléniques égaux ou différents ayant chacun 4 à 11 atomes de carbone.

3. Procédé de production de polyisocyanates de biuret dépourvus de monomère suivant la revendication 1, caractérisé en ce qu'on élimine par distillation ou par extraction les polyisocyanates dépourvus de groupes urée et biuret des solutions des polyisocyanates porteurs de groupes biuret dans des polyisocyanates dépourvus de groupes urée et biuret.

4. Procédé de production de polyisocyanates porteurs de groupes urée sous la forme dispersée dans des polyisocyanates dépourvus de groupes urée et biuret suivant la revendication 1, caractérisé en ce qu'on utilise comme polyisocyanate organique le 2,4-diisocyanato-toluène et/ou le 2,6-diisocyanatotoluéne et comme polyamine organique une diamine diprimaire aromatique portant éventuellement des substituents méthyle ou des ponts méthylène, totalisant 6 à 15 atomes de carbone, et en ce qu'on conduit la réaction dans la plage de temperatures de 20 à 120°C.

5. Dispersions stables de polyisocyanates porteurs de groupes urèe de formule:

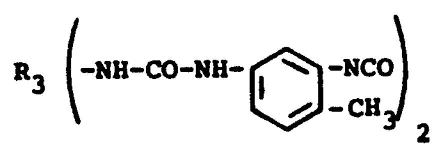

dans le 2,4- et/ou le 2,6-diisocyanatotoluène, $R_3$ représentant le reste divalent que l'on obtient en éliminant les groupes amino d'une diamine diprimaire aromatique éventuellement substituée par des groupes méthyle et/ou portant des ponts méthylène, totalisant 6 à 15 atomes de carbone.

## Claims

1. Process for the preparation of polyisocyanates containing urea groups and/or biuret groups dispersed or dissolved in polyisocyanates which are free from urea and biuret groups, by the reaction of organic polyisocyanates with organic polyamines containing at least 2 primary amino groups using an $NCO/NH_2$ equivalent ratio of at least 4:1, wherein the polyisocyanate used in excess which serves both as reactant and as solvent or dispersing agent is first introduced into the reaction vessel and the polyamine used in less than the equivalent amount, is introduced into the polyisocyanate in the reaction vessel at a temperature in the range of from −20 to 250°C, the preparation of the polyisocyanates containing biuret groups optionally taking place in two stages in such a way that a dispersion, initially formed at a relatively low temperature, of polyisocyanates containing urea groups is converted, by means of after-heating, into the solution of polyisocyanates containing biuret groups, characterised in that

a) the polyisocyanate component which is used in excess is introduced into a mixing chamber, the dimensions of which are such that the distance from the nozzle, mentioned in the following under b), to the internal wall of the mixing chamber measured in the direction of the injected jet is at least 100 times the diameter of the nozzle and the shortest distance from the nozzle jet to the lateral wall of the mixing chamber is at least 25 times the diameter of the nozzle, and

b) the polyamine component, which is used in less than the equivalent quantity is injected at a pressure of from 2 to 1000 bar and a relative velocity of at least 5 m/s into the polyisocyanate component in the reaction vessel by means of a straight jet nozzle which has an internal diameter of from 0.01 to 5 mm.

2. Process according to claim 1, characterised in that the organic polyisocyanate used is a diisocyanate of the formula

$$R_1(NCO)_2)$$

and the polyamine used is a diamine of the formula

$$R_2(NH_2)_2$$

in which;

$R_1$ and $R_2$ represent identical or different polymethylene groups having in each case from 4 to 11 carbon atoms.

3. Process for the preparation of monomer-free biuret polyisocyanates according to claim 1, characterised in that the polyisocyanates free from urea and biuret groups are removed from the solutions of the polyisocyanates containing biuret groups in polyisocyanates free from urea and biuret groups, by means of distillation or extraction.

4. Process for the preparation of urea-group-containing polyisocyanates, dispersed in polyisocyanates which are free from urea and biuret groups, according to claim 1, characterised in that the organic polyisocyanate used is 2,4-diisocyanatotoluene and/or 2,6-diisocyanatotoluene and the organic polyamine used is an aromatic diprimary diamine which may have methyl substituents or methylene bridges and which contains a total of from 6 to 15 carbon atoms and the reaction is carried out in the temperature range of from 20 to 120°C.

5. Stable dispersions of urea-group-containing polyisocyanates of the formula

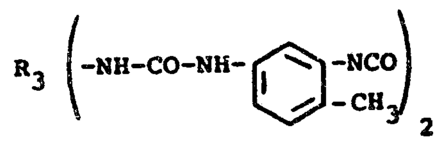

in 2,4 and/or 2,6-diisocyanatotoluene, in which formula $R_3$ represents a divalent radical such as is obtained by the removal of the amine groups from an aromatic diprimary diamine which has a total of from 6 to 15 carbon atoms and which may be methyl-substituted and/or contain methylene bridges.

ne

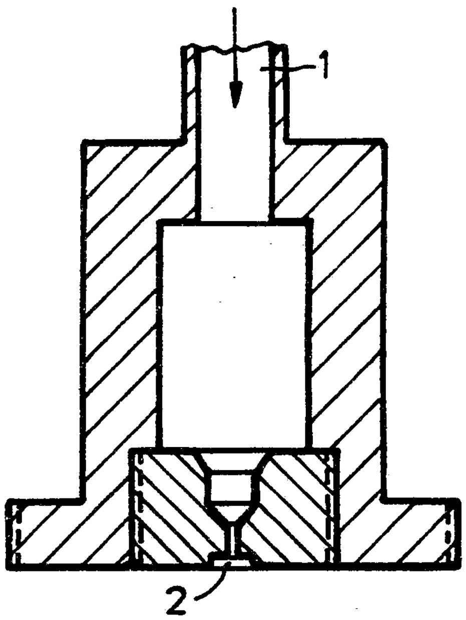


FIG. 1

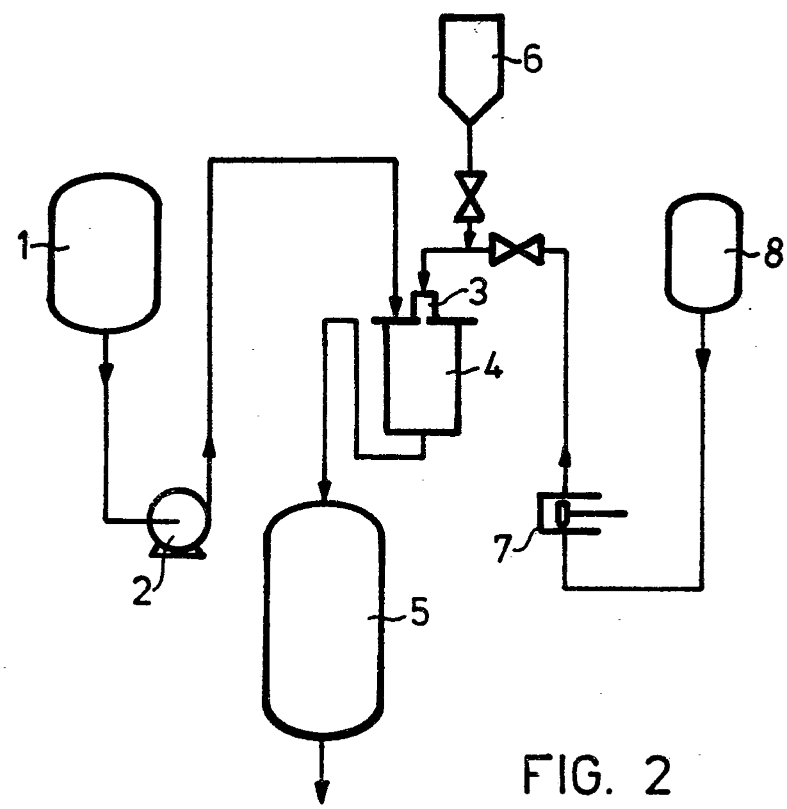


FIG. 2